# EUROPEAN PATENT APPLICATION

(11) **EP 4 537 821 A1**
(43) Date of publication of application: **16.04.2025**
(21) Application number: 23819285.0
(22) Date of filing: 12.06.2023
(51) Int. Cl.: A61K 31/18, A61K 31/075, A61K 31/135, A61K 31/185, A61P 35/00

(54) **USE OF SRE-PTPRZ1 INHIBITOR IN PREPARATION OF DRUG FOR TREATING SQUAMOUS CELL CARCINOMA**

(30) Priority: 10.06.2022 CN 202210654523
(71) Applicant: West China Hospital, Sichuan University, Chengdu, Sichuan 610041 (CN)
(72) Inventor: LI, Weimin, Chengdu, Sichuan 610041 (CN); XIE, Dan, Chengdu, Sichuan 610041 (CN); WANG, Zhoufeng, Chengdu, Sichuan 610041 (CN); XIA, Lin, Chengdu, Sichuan 610041 (CN); DENG, Yan, Chengdu, Sichuan 610041 (CN); PI, Xuenan, Chengdu, Sichuan 610041 (CN); WANG, Huan, Chengdu, Sichuan 610041 (CN); ZENG, Tianfu, Chengdu, Sichuan 610041 (CN)
(74) Representative: Altmann Stößel Dick Patentanwälte PartG mbB
(86) International application number: PCT/CN2023/099586
(87) International publication number: WO 2023/237122

(57) **Abstract**

A use of an SRE-PTPRZ1 inhibitor or a PTPRZ1 inhibitor in the preparation of a drug for treating squamous cell carcinoma. The SRE-PTPRZ1 inhibitor is an inhibitor for SRE-PTPRZ1 structural variation, the SRE-PTPRZ1 structural variation being somatic simple repeat expansion at insertion site GRCh38 chr7: 121603263-121603506, the repeating pattern of an insertion region and a somatic cell simple repeat expansion sequence are (CTTT)n, wherein 10 ≤ n < 500; the PTPRZ1 inhibitor is one of the followings compounds, a pharmaceutically acceptable salt thereof, one or two derivatives among derivatives thereof, or a mixture of the above: NAZ2329, SCB4380, MY-33-3, or MY-10; and the HGNC number of the PTPRZ1 gene is 9685.

## Description

### Field of the invention

The present invention relates to the field of *in vitro* diagnostic reagents, and specifically to the use of SRE-PTPTRZ1 inhibitors in the manufacture of medicaments for treatment of squamous cell carcinoma (SCC).

### Background of the invention

Squamous cell carcinoma (SCC) is a malignant tumor originating from the epidermis or skin appendages, commonly found in the skin, oral cavity, or mucosa, and can also originate from the bronchi, renal pelvis, and bladder. SCC presents a rapid progression, causes significant damage, and poses a serious threat to human health and life.

Lung squamous cell carcinoma (Lung-LUSC) is one of the most common lung cancers. At present, there are few treatment options available for the patients with Lung LUSC, and the progress of targeted therapy for this cancer type is slow. Therefore, finding key molecular targets that drive the progression of Lung LUSC is of great significance for its diagnosis and treatment.

Genetic mutations are an important factor leading to lung cancer. Gene mutation testing has become an important basis for guiding targeted therapy for advanced lung cancer patients in clinical practice. Gene mutations mainly include single nucleotide variations (SNVs) and structural variations (SVs) such as insertion/deletion, inversion, duplication, and ectopia of long fragments. In recent years, the next-generation sequencing (NGS) technology has revealed the association between some gene mutations (such as EGFR, KRAS, TP53, etc.) and lung cancer. NGS has the characteristics of long reads and high throughput, and can detect the structural variations of some SNVs, small INDELs, and small fragments in the genome, such as gene fusion and chromosomal rearrangement. The discovery of these new targets has benefited many lung cancer patients. However, the detection of complex and large SVs in tumor genomes still poses challenges, which are mainly due to the limitations in the short reads and GC preference of NGS, and especially repetitive sequences and insertion events.

Finding gene mutations associated with diseases such as lung cancer is expected to provide molecular targets for the diagnosis and treatment of related diseases, which is of great significance. Therefore, this remains a research topic of great concern for researchers in this field.

### Content of the invention

The objective of the present invention is to provide new therapeutic targets and biomarkers for SCC.

The technical solution of the present invention includes:
The use of SRE-PTPRZ 1 inhibitors in the manufacture of medicaments for the treatment of SCC.

Preferably, the medicaments are those for treating Lung LUSC.

Preferably, the SRE-PTPRZ1 inhibitors are the inhibitors of SRE-PTPRZ1 structural variation, which is a somatic SRE at insertion site GRCh38 chr7: 121603263-121603506, and the repeating pattern of an insertion region and a somatic SRE sequence are (CTTT)n, wherein 10 ≤ n < 500.

The present invention also provides the use of PTPRZ1 inhibitors in the manufacture of medicaments for the treatment of SCC.

Preferably, the medicaments are those for treating Lung LUSC.

Preferably, the PTPRZ1 inhibitors are selected from the group consisting of NAZ2329, SCB4380, MY-33-3, MY-10, or a pharmaceutically acceptable salt thereof, or a derivative thereof, or a mixture thereof.

Preferably, the PTPRZ 1 inhibitors are the inhibitors of the PTPRZ 1 gene, and the HGNC number of the PTPRZ 1 gene is 9685.

The present invention also provides a medicament for treating SCC, which is formed by using SRE-PTPRZ1 inhibitors or PTPRZ1 inhibitors as the active ingredient, in combination with pharmaceutically acceptable excipients or auxiliary ingredients.

Preferably, the medicament is that for treating Lung LUSC.

Preferably, the SRE-PTPRZ1 inhibitors are the inhibitors of SRE-PTPRZ1 structural variation, which is a somatic SRE at the insertion site GRCh38 chr7: 121603263-121603506, and the repeating pattern of an insertion region and a somatic SRE sequence are (CTTT)n, wherein 10 ≤ n < 500.

Preferably, the PTPRZ1 inhibitors are selected from the group consisting of NAZ2329, SCB4380, MY-33-3, MY-10, or a pharmaceutically acceptable salt thereof, or a derivative thereof, or a mixture thereof.

Preferably, the PTPRZ 1 inhibitors are the inhibitors of the PTPRZ 1 gene, and the HGNC number of the PTPRZ1 gene is 9685.

The present invention also provides the use of reagents for detecting the SRE-PTPRZ1 structural variation or the PTPRZ1 gene in the manufacture of SCC screening kits.

Preferably, the kit is a kit for screening Lung LUSC.

Preferably, the reagents for detecting the SRE-PTPRZ1 structural variation or the PTPRZ1 gene is a PCR detection reagent.

Preferably, the reagents for detecting the SRE-PTPRZ1 structural variation or the PTPRZ1 gene is a reagent for detecting the expression level of PTPRZ1 gene in tumor samples or blood samples.

Preferably, the SRE-PTPRZ1 structural variation involves somatic SRE at the insertion site GRCh38 chr7: 121603263-121603506, and the repeating pattern of an insertion region and a somatic SRE sequence are (CTTT)n, wherein 10 ≤ n < 500.

Preferably, the HGNC number of the PTPRZ1 gene is 9685.

The present invention also provides a screening kit for SCC, which comprises reagents for detecting the SRE-PTPRZ1 structural variation or the PTPRZ1 gene.

Preferably, the kit is a kit for screening Lung LUSC.

Preferably, the reagents for detecting the SRE-PTPRZ1 structural variation or the PTPRZ1 gene is a PCR detection reagent.

Preferably, the reagents for detecting the SRE-PTPRZ1 structural variation or the PTPRZ1 gene is a reagent for detecting the expression level of PTPRZ1 gene in tumor samples or blood samples.

Preferably, the SRE-PTPRZ1 structural variation involves the somatic SRE at the insertion site GRCh38 chr7: 121603263-121603506, and the repeating pattern of an insertion region and a somatic SRE sequence are (CTTT)n, wherein 10 ≤ n < 500.

Preferably, the HGNC number of the PTPRZ1 gene is 9685.

In the present invention, the structure of "NAZ2329" is: 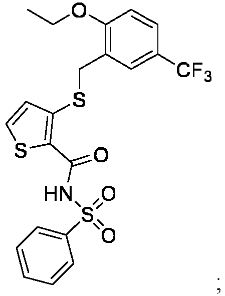 the structure of "SCB4380" is: 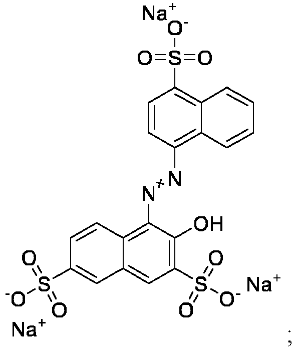 the structure of "MY-33-3" is: 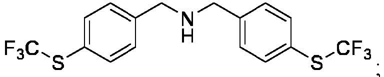 the structure of "MY-10" is:

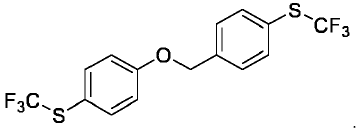

In the present invention, a somatic SV (SRE-PTPRZ1) is found with high frequency (41%) in Lung LUSC patients based on the whole genome sequencing data of lung cancer. This mutation is manifested as an insertion in the GRCh38 chr7:121603263-121603506 region of the human reference genome, which contains a SRE sequence with a repeating pattern of (CTTT)n (10 ≤ n < 500). Further research has indicated that the simple repeat expansion of somatic cells is associated with high expression of PTPRZ1. PTPRZ1 has only been reported in gliomas in the prior art, and it is generally believed that the main site of PTPRZ1 gene expression is in central nervous system tissues. There have been no reports on the association between PTPRZ1 gene and SCC such as Lung LUSC.

The key to the present invention lies in determining the presence of SRE-PTPRZ1 in SCC patients, and the significant difference in the expression level of PTPRZ1 gene compared to other types of cancer tissues.

Therefore, the medicaments targeting SRE-PTPRZ1 or PTPRZ1 (SRE-PTPRZ1 inhibitors, PTPRZ1 inhibitors) are expected to be used as therapeutic drugs for SCC. In addition, SCC screening can be performed by detecting the expression level of PTPRZ1 in tumor tissue or blood. As for the specific means of detecting the expression level of PTPRZ1 gene, various available techniques in the prior art can be used. In the examples of the present invention, the detection is carried out by specific PCR method, but not limited to it. Any method that can detect the expression level of PTPRZ1 gene can be used for screening SCC.

The present invention provides a new SCC screening marker and a new SCC screening kit, which can achieve effective screening of SCC. The present invention has good application prospects.

Obviously, based on the above content of the present invention, according to the common technical knowledge and the conventional means in the field, other various modifications, alternations, or changes can further be made, without department from the above basic technical spirits.

With reference to the following specific examples, the above content of the present invention is further illustrated. But it should not be construed that the scope of the above subject matter of the present invention is limited to the following examples. The techniques realized based on the above content of the present invention are all within the scope of the present invention.

### Description of Figures

Figure 1: Hierarchical clustering of Lung LUSC and lung adenocarcinoma (Lung LUAD) samples based on a somatic simple repeat expansion shared by more than three samples using whole genome sequencing. The hierarchical clustering is based on the Jaccard distance between samples. Cluster 2 (cluster_2) members are all Lung LUSC samples.

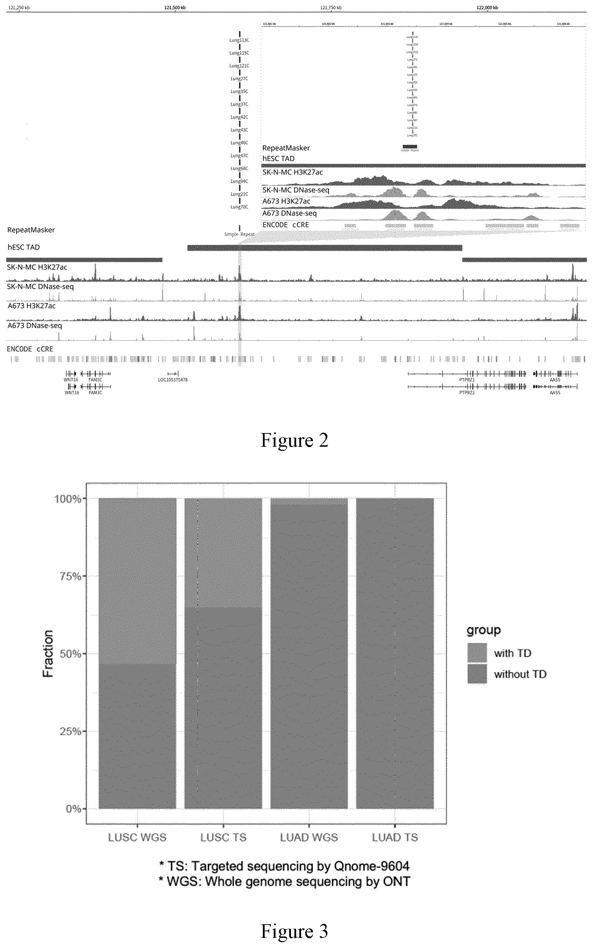

Figure 2: Insertion positions of somatic simple repeat expansion in the human reference genome shared by 12 Lung LUSC samples. The annotation from top to bottom in the figure shows the insertion positions of somatic simple repeat expansion in 12 Lung LUSC samples. RepeatMasker annotation: human embryonic stem cell Hi-C data predicting topologically associating domain (hESC TAD), SK-N-MC cell lines ChIP Seq H3K27ac predicting enhancers, SK-N-MC cell lines DNase-seq predicting regulatory regions, A673 cell lines ChIP Seq H3K27ac predicting enhancers, A673 cell lines DNase seq predicting regulatory regions, ENCODE cCRE, GENCODE gene annotation.
Figure 3: The proportion of samples with simple repeat expansion (SRE) detected in Lung LUSC and lung adenocarcinoma samples. TS: Targeted sequencing; WGS: Whole genome sequencing.
Figure 4: The above figure shows the expression level of PTPRZ1 gene in GTEX human tissue; the figure below shows the expression level of PTPRZ1 gene in samples of non-small cell lung cancer (85 cases) with or without the somatic simple repeat expansion based on whole genome sequencing. With SRE: There is the somatic simple repeat expansion; without SRE: There is not the somatic simple repeat expansion.
Figure 5: The expression level of PTPRZ1 gene in TCGA samples and the expression level of PTPRZ1 gene in non-small cell lung cancer samples with or without the somatic simple repeat expansion based on the whole genome sequencing. With SRE: There is the somatic simple repeat expansion; without SRE: There is not the somatic simple repeat expansion.
Figure 6: Experimental results of PTPRZ1 inhibitor NAZ2329 against different Lung LUSC cell lines.
Figure 7: Experimental results of PTPRZ1 inhibitor MY33-3 against different cancer cell lines.
Figure 8: Animal experiment results of PTPRZ1 inhibitor MY33-3 as a therapeutic drug for squamous cell carcinoma.
Figure 9: Experimental results of PTPRZ1 inhibitor MY-10 against different Lung LUSC cell lines.

### Examples

### Example 1 The relationship between the expression level of PTPRZ1 gene and Lung LUSC

### I. Sample inclusion

This study included 105 cases of SCC and 161 cases of adenocarcinoma diagnosed by pathology department at West China Hospital of Sichuan University, of which 124 samples were assigned to the experimental group. Amongst, ONT and WGS were used to sequence tumor tissue and adjacent tissues, while RNA was only used to sequence tumor samples. 132 samples were selected as the validation group, and Qnome-9604 was mainly used for the target region sequencing for the upstream of PTPRZ1. Specific sequencing plan:

| Sequencing type | ONT | WGS | RNA | Qnome-9604 (Additional samples) |
|---|---|---|---|---|
| Adenocarcinoma | 101 | 101 | 63 | 50 |
| SCC | 23 | 23 | 13 | 82 |

### II. Sample processing

Each sample contains both tumor tissues and distal normal tissues, respectively. The collection and cutting of specimens were performed by pathologists from the Pathology Department of West China Hospital. The removed tissue specimens were placed in centrifuge tubes containing 50 mL of Hank's Balanced Salt Solution (HBSS) and rapidly transferred to the laboratory at low temperature. Hemostatic forceps were used to remove the tumor from the BD tube, and then the necrotic tissue on the surface was cut off. The tissue was cut into small pieces using scissors, and then cut into minced meat using ophthalmic scissors. This process was performed on ice. Two clumps of minced meat were taken out and placed in two cryotubes. Both tubes were stored at -80 °C. The tissues would be used for WGS and RNA sequencing on Illumina platform, as well as WGS sequencing on ONT platform.

### III. Test procedures

### 1. WGS sequencing on Illumina platform

DNA from tumors, adjacent tissues, or blood samples was extracted with the DNeasy Blood & Tissue Kit (QIAGEN, Hilden, Germany). 0.4 µg of qualified genomic DNA was randomly broken into fragments of approximate 350 bp in length using a Covaris breaker. After terminal repair, phosphorylation, and adding A-tail, the Illumina sequencing adapter was ligated to both ends of the library DNA using T4 DNA ligase. For libraries with added adapters, the Agencourt SPRIselect nucleic acid fragment screening kit was used to purify and screen the library: first, the original library with appropriate fragment length was screened using SPRI magnetic beads for the next step of PCR amplification; the library was further purified to remove sequencing adapters and the linked products of adapters themselves. After the construction of the library, Qubit 2.0 was used for initial quantification, followed by detecting the length of the inserted fragments in the library with Agilent 2100. After the length of the inserted fragments met expectations, qPCR method was used to accurately quantify the effective concentration (3 nM) of the library to ensure its quality. After the library inspection was qualified, Illumina Novaseq PE150 sequencing was performed based on the effective concentration and data output requirements of the library. PE150 (Pair End 150 bp) referred to high-throughput paired-end sequencing, with each end measuring 150 bp.

### 2. RNA sequencing on Illumina platform

RNA from tumor samples and normal control samples was extracted using Trizol. The quality of the extracted RNA was strictly controlled: (1) RNA degradation and contamination were detected with agarose gel at a concentration of 1%; (2) Nanodrop and Qubit were respectively used to detect RNA purity and concentration; (3) Agilent 2100 Bioanalyzer was chosen to accurately detect RNA integrity. The library of each sample was constructed using 1 µg of RNA. mRNA with polyA tails was enriched using Oligo (dT) magnetic beads, and then the obtained mRNA was randomly broken with divalent cations in Fragmentation Buffer. Using fragmented mRNA as a template and random oligonucleotides as primers, the first strand of cDNA was synthesized in the M-MuLV reverse transcriptase system. Subsequently, the RNA strand was degraded using RNaseH, and the second strand of cDNA was synthesized from dNTPs in the DNA polymerase I system. After purification, terminal repair, and adding A, adapter ligation was performed to screen library fragments with lengths ranging from 370 bp to 420 bp. Then, PCR was carried out to add index tags and enrich the library. Finally, the length of the inserted fragments in the library was analyzed using the Bioanalyzer 2100 system, and paired-end 150 bp sequencing (PE150) was finished on the Illumina Novaseq platform.

### 3. WGS sequencing on ONT platform

Complete DNA was extracted from the sample using QIAGEN Genomic tip 100/G (QIAGEN, Hilden, Germany). 8 µg of qualified genomic DNA was randomly broken into fragments with a length of about 30 kb using g-TUBE, and then DNA fragments with a length of over 15 kb were screened using Bluepippin. The purified DNA fragments were used to construct a library using Ligation Sequencing Kit (SQK-LSK109, ONT): first, after terminal repair, phosphorylation, and adding A-tail, the reaction was performed using ligase. After further purification, the standard sequencing adapter and motor protein obtained from ONT were ligated; the library was further purified to remove sequencing adapters. Sequencing was performed on PromethION platform based on the effective concentration and data output requirements of the library.

### 4. ONT WGS sequence processing

The whole genome sequence obtained from ONT platform was aligned with the human reference genome GRCh38 using NGMLR (version 0.2.8), with parameters -t 40 -x ont.

### 5. Sequencing of PCR capture products on the QiTan Platform

Qubit fluorescent quantitative instrument and agarose gel electrophoresis were used to analyze DNA concentration and fragment length. The ligation sequencing kit from QiTan Tech. was used for library sequencing: first, after terminal repair, phosphorylation, and adding A-tail, the QiTan barcode sequence was linked; after purification, an equal amount of nucleic acids ligated with QiTan barcode was taken out and mixed, followed by linking to the QiTan sequencing adapter; the library was purified to remove excess sequencing adapters. The library was quantified with Qubit, and then QNome-3841 platform was used for sequencing based on the effective concentration and data output of the library.

### 6. Detection of somatic structural variation based on whole genome sequencing

Based on the reference genome alignment results of DNA long sequences with ONT whole genome, structural variation detection was performed using Sniffles (version 1.0.11), and the parameters were set to support a minimum sequence number of 5 and a minimum length of 30 bp for structural variation. The reported structural variations with a length of less than 50 bp were further eliminated. The detection includes five types of structural variations: insertion, deletion, replication, inversion, and translocation. Structural variations were grouped according their types for analysis and merging, as well as more precise breakpoint locations were detected. The specific procedures are as follows:
1) The distance between two adjacent structural variations of tumor samples, as reported by Sniffles, was calculated to obtain a distance matrix. If the distance between adjacent structural variations is less than the tenth percentile of the distance matrix, the two adjacent structural variations will be merged. 2) The split-alignment-based (SAB) sequence information of various structural variations reported by Sniffles was extracted, including chromosome number, breakpoint position, and sequence position. A SAB sequence is a sequence to be sequenced, with one portion aligned to a certain position in the genome and the other portion aligned to other positions in the genome. The region between the maximum alignment-supporting breakpoints and the breakpoints reported by Sniffles was defined as the candidate breakpoint region. 3) If there is no significant difference in the numbers of SAB sequences located in the candidate breakpoint regions of the tumor and the corresponding blood sample by statistical test (Kolmogorov-Smirnov test, p value < 0.05), this structural variation will be removed from the somatic structural variation result. 4) If there is no significant difference in the size of structural variations located in the candidate breakpoint regions of both the tumor and the corresponding blood sample by the statistical test (Wilcoxon rank-sum test, p value < 0.05), this structural variation will be removed from the somatic structural variation result. 5) The filtered structural variations were manually screened based on SAB conditions using the Integrative Genomics Viewer.

### 7. Assembly of somatic structural variations based on whole genome sequencing

After completing the identification of somatic structural variations, the assembly of somatic structural variations was carried out by the following steps. 1) The supporting sequences for structural variations were extracted based on Sniffles results. 2) The supporting sequences were assembled using Shasta (version 0.5.1) and the following parameters: Reads. minReadLength (1000), MinHash.minHashIterationCount (60), MinHash.minFrequency (2), Align.minAlignedMarkerCount (100), and MarkerGraph.minCoverage (5). 3) After obtaining the structural variation assembly draft, Racon (version 1.4.3) was used to perform 4 rounds of iterative polishing according to the supporting sequences. Then, further polishing was carried out using the Medaka (version 1.0.3) r941_prom_high_g360 model. 4) The polished assembly results was aligned with the human reference genome GRCh38 using minimap2, to obtain accurate breakpoint positions.

### 8. Annotation of somatic insertion and replication based on whole genome sequencing

The assembled somatic insertion and replication were annotated using RepeatMasker (version 4.1.0), along with 50 bp reference genome sequences in its upstream and downstream, and annotations were further added using TRF (version 4.10.0) and Sdust. All regions that had not been annotated as transposons by RepeatMasker were re-annotated using TRF. All regions annotated as Unmask by RepeatMasker and longer than 5 bp were re-annotated using TRF and Sdust. According to the length, TRF annotation results could be classified into simple repeats (shorter than 10 bp), tandem repeats (between 10 bp and 100 bp), and satellite repeats (longer than 100 bp).

### 9. Recognition of somatic insertion and replication based on whole genome sequencing

All assembled somatic insertion/replication sequences were classified into two categories: somatic tandem replication and somatic insertion. The differentiation method was to compare the assembled sequence with the reference genome sequence around the insertion breakpoint in a whole fragment or repetitive pattern. In the whole fragment pattern, the reference genome sequence for the upstream and downstream of the insertion/replication sequence was captured, with a capture length of the assembly sequence length + 50 bp. The captured sequence was compared with the corresponding insertion/replication sequence using Needle (EMBOSS version 6.6.0.0), and similarity values were calculated. The insertion/replication sequences with similarity values of >90%, compared to the reference genome sequence, were defined as somatic tandem replication. The repetitive pattern mainly targeted insertion/replication sequences in which >90% of the regions were annotated by TRF and only one repetitive pattern was found. For this type of sequence, the reference genome sequences for the upstream and downstream of the insertion/replication sequence were captured, with a capture length of 1.5 times the length of the insertion/replication sequence from the repetitive pattern plus 10 bp. The paired local alignment (Gotoh local alignment) was used to compare the insertion/replication sequences from repeat patterns and the captured reference genome sequences. In the repetitive pattern, the insertion/replication sequences with similarity values of >85%, compared to the captured reference genome sequence, were defined as somatic tandem replication. All insertion/replication sequences that could not be defined as somatic tandem replicators were classified as somatic insertions.

### 10. Recognition of target SRE based on PCR capture sequencing

The QiTan third-generation sequencing data with a length of >500 bp and a quality of >7 were retained, and then minimap2 was used to align the captured sequencing data to the hg38 reference genome. Then, the alignment results in the chr7: 121603000-121604000 region of the reference genome were extracted. The number of reads containing a length of not less than 50 bp and 75% of the inserted sequences in the specified pattern in this region was counted. The proportion of reads meeting the above conditions, based on all reads in the specified region, was calculated. If the proportion was not less than 0.6%, the sample was that carrying the somatic SRE.

### 11. Analysis of spatial proximity of SRE and PTPRZ1 genes in cell lines containing SRE sequences based on 3C-PCR

3C library construction: HCC-95 and H226 cell lines were used for 3C experiment. 1 × 10⁷ cells were allowed to cross-link with formaldehyde for 10 min, and then quenched with glycine (with a final concentration of 0.125 mM) at room temperature for 5 min. Using 500 µL of 1x cold lysis buffer (10 mM Tris-HCl pH=8.0, 10 mM NaCl, 0.2% Igecal CA630) containing 1 x protease inhibitor (Roche, Indianapolis, IN, USA), cell counting and lysis were carried out on ice for at least 15 min. The cell nucleus was made into granules, washed with 500 µL of cold 1× MseI buffer (NEB, Ipswich, MA, USA), and re-suspended in 500 µL of 1× MseI buffer containing 0.3% SDS, followed by incubating at 37 °C for 1 h. Then, 1% Triton X-100 was added to the sample, and the sample was further incubated for 1 h. Each sample was digested overnight at 37 °C using 600 U restriction endonuclease. On the second day, SDS was added at a final concentration of 1.6%, and the sample was incubated at 65 °C for 20 min to stop digestion. The reaction was carried out at 16 °C for 4 h, using the following system: 745 µL of 10×T4 ligase buffer, 10% Triton-X 100, 80 µL of 10 mg/mL BSA, 6 mL of water, 575 µL of cell lysate, and 10 U T4 ligase (Invitrogen). De-crosslinking was performed with proteinase K (Invitrogen) overnight at 65 °C. Finally, the 3C sample was purified by extraction with phenol-chloroform, and quantified using Qubit dsDNA HS Assay (Life Technologies).

3C-PCR: Due to SRE being a repetitive sequence, primers cannot be designed; therefore, primers were designed in the enhancer region on both sides of the SRE insertion, to detect the spatial relationship between PTPRZ1 and the enhancer region of SRE insertion. Anchor primer was designed near PTPRZ1. The test primer was designed around the MseI cleavage sites near enhancer 1 and enhancer 2. Each test primer was paired with an anchor primer. The primer sequence was shown in Supplementary Table S8. All PCR was performed using PremSTAR Max Premix (2x) (Takara R045). The system was as follows: For each 50 µL reaction: 25 µL of 2× PremSTAR Max Premix, 0.8 µL of 10 µM anchor primer, 0.8 µL of 10 µM test primer, and 100 ng of 3C DNA. PCR was performed as follows: 98 °C, 2 min; 98 °C, 10 sec; 60 °C, 15 sec; 72 °C, 60 sec; repeating 30 times, and finally, the sample was extended at 72 °C for 2 min. PCR products were purified (Invitrogen) and sequenced (QiTan Tech., Beijing, China).

### 12. Detection of the regulatory effect of CFCT protein on the spatial proximity of SRE and PTPRZ1 genes based on a dual-luciferase reporter assay system

Two enhancer sequences (named enhancer1 and enhancer2) on both sides of the SRE insertion site, PTPRZ1 promoter sequence, and predicted CTCF binding site sequence were cloned into the pGL3 basic vector, and the CTCF gene was cloned into the pcDNA3.1 basic vector. The constructed positive plasmid was confirmed by DNA sequencing. HEK293T cells were cultured in a 6-well plate until 80% fusion and then transfected with the reporter plasmid. Cells were transfected with 200 ng of enhancer 1, enhancer 2, or PTPRZ1 promoter-luciferase reporter vector and 10 ng of Renilla luciferase plasmid (pRL TK), pcDNA3.1-CTCF, or control plasmid, and then incubated for 24-48 h. According to the manufacturer's protocol, the dual luciferase reporter system detection kit (Promega) was used to measure firefly luciferase activity and Renilla luciferase activity, and the relative luciferase activity was determined by standardizing firefly luciferase activity and Renilla luciferase activity. All experiments were repeated three times.

### IV. Experimental results

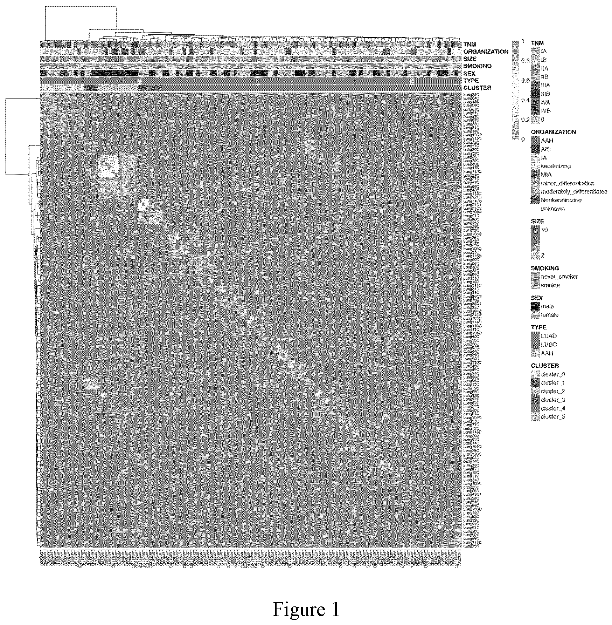

As shown in Figure 1, among 124 non-small cell lung cancer (NSCLC) samples that underwent whole genome sequencing (23 cases of Lung LUSC, 99 cases of lung adenocarcinoma, and 2 cases of atypical adenomatous hyperplasia), Jaccard distance was calculated between samples using somatic tandem replication shared by more than 3 samples. The samples were hierarchically clustered according to Jaccard distance, to obtain a special cluster 2, which had 12 samples and were all Lung LUSC samples. Cluster 2 could be capble of clustering because all members shared a somatic SRE at the insertion site GRCh38 chr7: 121603431-121603435, which is annotated as a simple repeat region by RepeatMasker (Figure 2). The repeat pattern of the insertion region and the somatic SRE was (CTTT)n. One Lung LUSC sample and two lung adenocarcinoma (Lung LUAD) samples located outside cluster 2 were also detected to have the somatic SRE. The length of this somatic SRE detected in the sample was 172-545 bp (median 308 bp).

In order to verify the feasibility of the somatic SRE as a key target in Lung LUSC, targeted sequencing was performed on additional 50 Lung LUAD samples, 60 Lung LUSC samples, 28 small cell lung cancer (SCLC) samples, and their paired blood samples on the Qnome-9604 platform. The somatic SRE was detected in 21 Lung LUSC samples and 12 SCLC samples, and was not detected in Lung LUAD samples. In addition, 22 pairs of Lung LUSC and paired blood samples were sequenced on the PromethION platform. Among them, 12 Lung LUSC samples were detected to have the somatic SRE (Figure 3). Based on all sequencing samples, the somatic SRE was detected in 41% of sequencing Lung LUSC samples.

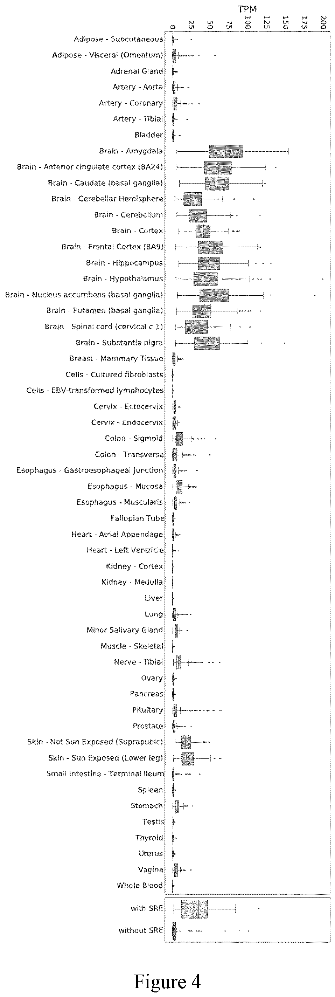

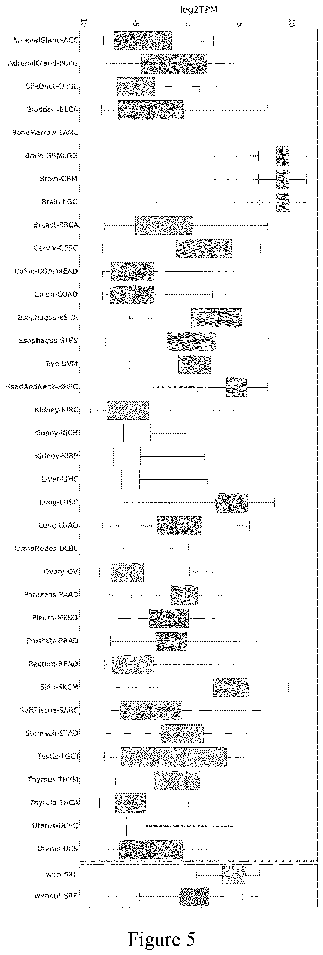

For genes located within 500 kb upstream and downstream of the insertion site of the somatic SRE, the gene expression levels were compared between samples with and without this somatic SRE (For 85 cases with RNA-Seq data, the samples with the somatic SRE:the samples without the somatic SRE = 75:10), and only the PTPRZ1 gene showed significant differences (Wilcoxon rank-sum test, p=3.92e-5, log2|fold change|=2.34). PTPRZ1 was previously only reported to be found in glioma, and its gene expression was also believed to be mainly in central nervous system tissues. However, in NSCLC samples with the insertion of the somatic SRE, the expression level of PTPRZ1 reached its high expression level in normal human brain tissue (Figure 4). In the TCGA data, there was a statistically significant difference in the expression level of PTPRZ1 RNA between lung squamous cell carcinoma (Lung LUSC) and lung adenocarcinoma (Lung LUAD) (adjusted p value = 6.74e - 162, log2|fold change|=3.36, Figure 5).

Among the cancer types included in TCGA, except for brain tumors (Brain-GBMLGG, Brain-GBM, Brain-LGG), skin melanoma (Skin-SKCM), lung squamous cell carcinoma (Lung-LUSC), and head and neck cancer (HeadAndNeck-HNSC), PTPRZ1 was highly expressed (Figure 5). These three types of cancer are either SCC or mainly classified as SCC, indicating the important role of PTPRZ1 in the development of SCC.

The above results indicated that the presence of somatic SRE (SRE-PTPRZ1 structural variation) was significantly associated with SCC, and the somatic SRE lead to the high expression of PTPRZ1 gene. In addition, the expression level of PTPRZ1 gene varied significantly among patients with different cancers, and SCC patients showed significantly higher expression levels of PTPRZ1 gene. Therefore, screening for SCC, including Lung-LUSC, could be performed by detecting SRE-PTPRZ1 structural variations or the expression level of PTPRZ1 gene in the cancer tissues of patients. Meanwhile, SRE-PTPRZ1 structural variations or PTPRZ1 gene were also expected to become therapeutic targets for SCC.

### Example 2 PTPRZ1 inhibitor NAZ2329 as a therapeutic drug for SCC

### 1. Experimental methods

The CCK8 method was used to detect the inhibition rate of PTPRZ1 inhibitor NAZ2329 on cell growth. 5 strains of human Lung-LUSC cells, one strain of human normal lung epithelial cell BEAS-2B, and one strain of highly expressed PTPRZI cell line U251 (High expression of PTPRZI gene in glioma cells) were used in this experiment for detection. Each cell line was inoculated into a 96-well plate at a density of 8000 cells/well, and cultured for 18 h. Then, 100 µL of NAZ2329 solution was added to the test wells at final concentrations of 5 µM, 10 µM, 20 µM, 30 µM, 40 µM, 50 µM, 60 µM, and 70 µM, while 100 µL of medium was added to the blank control well. After 72 hours of drug treatment, 20 µL of CCK8 was added in proportion and incubated for 2 h. The absorbance value was measured at 450 nm and the inhibition rate curve was plotted.

### 2. Experimental results

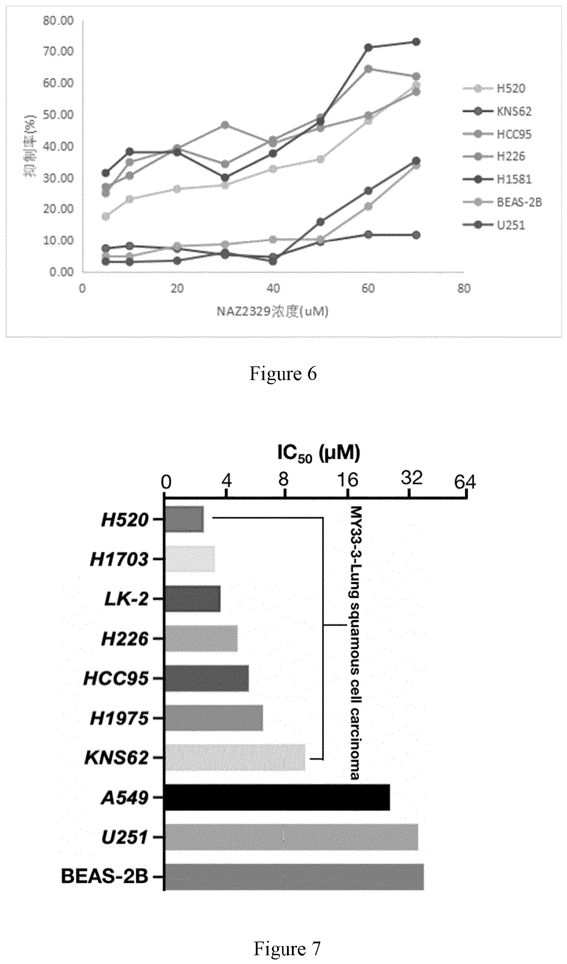

As shown in Figure 6, the inhibition rate of PTPRZ1 inhibitor NAZ2329 on cell growth was detected using 7 cell lines (5 strains of human Lung-LUSC cells, one strain of human normal lung epithelial cell BEAS-2B, and one strain of PTPRZI glioma cell line), and blank wells were used as the controls to calculate the inhibition rate on cell growth when treated at different concentrations. From Figure 6, it could be found that with the increase of NAZ2329 treatment concentration, the growth of all cell lines was inhibited, and the growth inhibition rate on 4 strains of Lung-LUSC cells was significantly higher than that of human normal lung epithelial cells BEAS-2B and glioma cell line U251, indicating that the PTPRZ1 inhibitor NAZ2329 had the potential to be used as a therapeutic drug for SCC.

### Example 3 PTPRZ1 inhibitor MY33-3 as a therapeutic drug for SCC

### 1. Experimental methods

The CCK8 method was used to detect the effect of PTPRZ1 inhibitor MY33-3 on cell proliferation. 7 strains of human Lung-LUSC cells, one strain of human lung adenocarcinoma cell, one strain of human normal lung epithelial cell BEAS-2B, and one strain of PTPRZI high expressing cell line U251 (high expression of PTPRZI gene in glioma cells) were used in this experiment for detection. Each cell line was inoculated into a 96-well plate at a density of 6000 cells/well, cultured for 24 hours, and then 100 µL of MY33-3 working solution was added to the test wells at final concentrations of 0 µM, 1 µM, 2 µM, 4 µM, 8 µM, 16 µM, 32 µM, and 64 µM, while 100 µL of medium was added to the blank control well. After 72 hours of drug treatment, 20 µL of CCK8 was added in proportion and incubated for 1 hour. The absorbance value was measured at 450 nm, and the IC₅₀ values of MY33-3 against different cells were plotted.

### 2. Experimental results

As shown in Figure 7, the effect of PTPRZ1 inhibitor MY33-3 on cell proliferation was detected using 10 cell lines (7 human Lung-LUSC cell lines, one human lung adenocarcinoma cell line, one human normal lung epithelial cell line BEAS-2B, and one PTPRZI glioma cell line), and blank wells were used as controls to calculate the IC₅₀ values against cells treated with different concentrations. From Figure 7, it could be found that the IC₅₀ values of 7 human Lung LUSC cell lines were all below 16 µM, while the IC₅₀ values of the remaining one human Lung LUAD cell line, one human normal lung epithelial cell line BEAS-2B, and one PTPRZI glioma cell line are 25 µM, 28 µM, and 33 µM, respectively. Moreover, MY33-3 has a better inhibitory effect on 7 human Lung-LUSC cell lines than three other cell lines, indicating the PTPRZ1 inhibitor MY33-3 was expected to be used as a therapeutic drug for SCC.

### Example 4 PTPRZ1 inhibitor MY33-3 as a therapeutic drug for SCC (animal experiment)

### 1. Experimental methods

At the animal level, the method of subcutaneous tumor formation in nude mice was used to verify the therapeutic effect of the inhibitor MY33-3 on the tumor formation of human Lung-LUSC (H520) cells. Firstly, 30 of 4W BALB/c-nu mice were purchased from GemPharmatech and acclimated for 3 days. Then, 150 µL of pre-prepared H520 cells was injected subcutaneously into the right forelimb of nude mice at 8x10⁶ cells/mouse. After 7 days, the tumor growth of each nude mouse was observed, and the mice were grouped and received drugs when the tumor grew to 100 mm³. Mice were divided into the blank group, the model group, the low-dose group, and the high-dose group, with 7 mice in each group. Meanwhile, the inhibitor MY33-3 was administered at doses of 20 mg/kg (low-dose group) and 100 mg/kg (high-dose group) once every 2 days, and the changes in tumor growth were recorded using a vernier caliper. Then, after 21 days of administration, samples were collected, and the therapeutic effect of the inhibitor MY33-3 on human Lung-LUSC cells was evaluated using indicators such as tumor volume, tumor weight, and nude mouse weight.

### 2. Experimental results

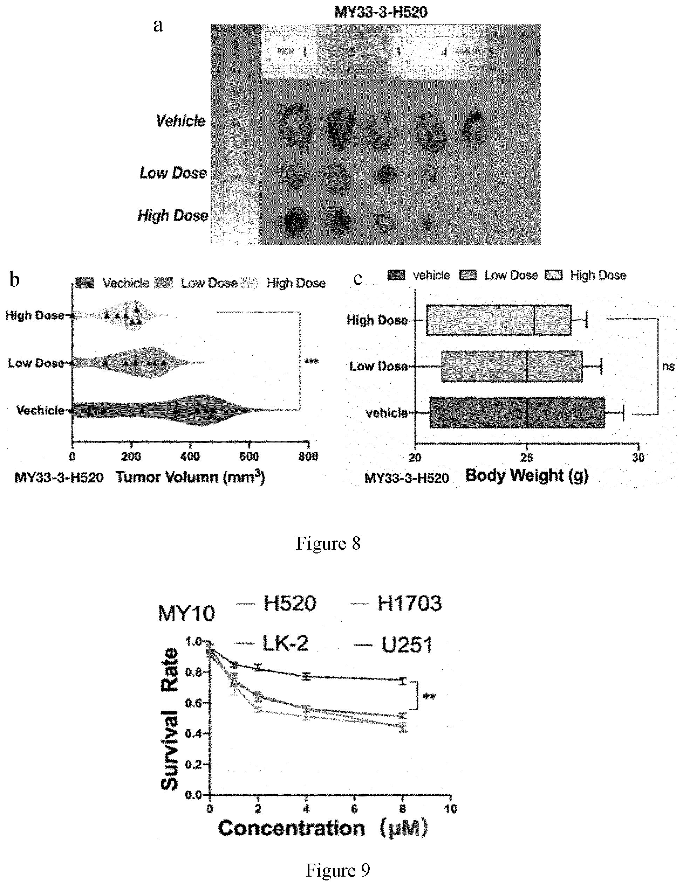

As shown in Figure 8a, compared with the model group, after 21 days of administration, the tumor size was significantly decreased in the low-dose and high-dose groups of the inhibitor MY33-3. Moreover, the inhibitor MY33-3 had a good inhibitory effect on the tumorigenicity of human Lung-LUSC cells (H520) at a dose of 20 mg/kg. Figure 8b shows the calculation results of tumor volume for the model group, low-dose group, and high-dose group. It was shown that compared with the model group, after 21 days of administration, the tumor volume was more significantly decreased in the low-dose and high-dose groups of the inhibitor MY33-3. Figure 8c shows the trend of weight gain in nude mice during the experimental procedures. It was demonstrated that the weight of the model group, low-dose group, and high-dose group showed a significant increase trend, and there was no significant difference in the weight of nude mice among the model group, low-dose group, and high-dose group. This indicated that the doses of 20 mg/kg and 100 mg/kg did not have any adverse effects on the weight gain of nude mice. From the perspective of animal experiments, it was indicated that the PTPRZ1 inhibitor MY33-3 had the potential to be used as a therapeutic drug for SCC.

### Example 5 PTPRZ1 inhibitor MY-10 as a therapeutic drug for SCC

### 1. Experimental methods

The CCK8 method was used to detect the effect of the PTPRZ1 inhibitor MY-10 on cell proliferation. 3 human Lung-LUSC cells and one PTPRZI high expression cell line U251 (high expression of PTPRZI gene in glioma cells) were used in this experiment for detection. Each cell line was inoculated into a 96-well plate at a density of 5000 cells/well, cultured for 24 hours, and then 100 µL of MY-10 working solution was added at final concentrations of 0 µM, 1 µM, 2 µM, 4 µM, 6 µM, and 8 µM to the test wells, while 100 µL of medium was added to the blank control well. After 72 hours of drug treatment, 20 µL of CCK8 was added in proportion, followed by incubating for 1 h. The absorbance value was measured at 450 nm, and the survival rates of MY-10 against different Lung-LUSC cells were plotted.

### 2. Experimental results

As shown in Figure 9, the effect of the PTPRZ1 inhibitor MY-10 on cell growth and proliferation was detected using 4 cell lines (3 human Lung-LUSC cell lines and one PTPRZI glioma cell line), and the blank wells were used as the controls to calculate cell survival rates under different concentrations of treatment. From Figure 9, it was shown that the survival rate of the three human Lung-LUSC cells showed a decreasing trend with the increase of MY-10 inhibitor concentration, and the most significant effect was on the survival rate of H520 cells, with an IC₅₀ value of about 4 µM. Meanwhile, MY-10 inhibitors also had a certain effect on the survival rate of the highly expressed cell line U251 (high expression of PTPRZI gene in glioma cells). The MY-10 inhibitor showed significant inhibitory effects on three human Lung-LUSC lines (H520, H1703, LK-2) and one PTPRZI glioma cell line (U251) with increasing concentrations. The PTPRZ1 inhibitor MY33-3 was expected to be used as a therapeutic drug for SCC.

### Example 6 Composition and usage method of detection kit 1 (detecting SRE insertion) of the present invention

### 1. Composition of the kit

The composition of the detection kit was the reaction system for PCR amplification, which was as follows:

| | |
|---|---|
| Nuclease-free H₂O | 10 µL |
| 2.5 µM PCR Primer 1(SEQ ID NO. 1) | 2.5 µL |
| 2.5 µM Barcoded PCR Primer 2 (Corresponding to different primers, SEQ ID NO. 2) | 2.5 µL |
| NEB Next High-Fidelity 2× PCR Master Mix | 25 µL |

The primer sequences used for PCR in the detection kit were as follows:
Primer 1 (SEQ ID NO. 1): CAGCCAGTAAAAATGACTTCAGAGC
Primer 2 (SEQ ID NO. 2): AGAGCTGCAGCAAGACTCG

### 2. Instructions for the kit

DNA extraction: Using DNeasy Blood & Tissue Kit (QIAGEN, Hilden, Germany) to extract DNA from tumors, paracancerous tissues, or blood samples.

Sequencing of PCR product: Following the steps of "Sequencing of PCR capture products on the QiTan Platform" in Part III of Example 1.

Recognition of target SRE: Following the steps of "Recognition of target SRE based on PCR capture sequencing" in part III of Example 1.

### Example 7 Composition and usage method of detection kit 2 (detecting the expression level of PTPRZ1) of the present invention

### 1. Composition of the kit

The composition of the detection kit was the reaction system for qPCR amplification, which was as follows:

| | |
|---|---|
| Nuclease-free H₂O | 8.6 µL |
| 10 µM qPCR Primer 3 (SEQ ID NO. 3) | 0.2 µL |
| 10 µM qPCR Primer 4 (SEQ ID NO. 4) | 0.2 µL |
| cDNA | 1 µL |
| SYBR Master mix | 10 µL |

The primer sequences used for qPCR in the detection kit were as follows:
Primer 3 (SEQ ID NO. 3): TGCGAATCCTAAAGCGTTTCC
Primer 4 (SEQ ID NO. 4): TCCATTAGCCCAATCCAGGC

### 2. Instructions for the kit

RNA extraction and cDNA generation: RNA was extracted from tumor, paracancerous tissues or blood samples using Triol method. After determination of the concentration using qubits, equal amounts of RNA was used to perform reverse transcription to generate corresponding cDNA with PrimeScript^{™} RT reagent kit (Perfect Real Time) (Takara, Beijing, China).

qPCR detection: The expression level of PTPRZ1 gene in the sample was quantified using SYBR Green gPCR Master Mix (MedChemExpress, Shanghai, China).

According to the above examples, it was first discovered in the present invention that the somatic simple repeat expansion of SRE-PTPRZ1 was correlated with SCC, and SRE-PTPRZ1 could lead to high expression of PTPRZ1. The above findings provided new targets and biomarkers for the treatment and detection of SCC, which could be used for the treatment and auxiliary diagnosis of cancer in clinical practice, with good clinical application prospects.

## Claims

1. The use of SRE-PTPRZ 1 inhibitors in the manufacture of medicaments for the treatment of squamous cell carcinoma (SCC).

2. The use according to claim 1, **characterized in that** the medicaments are those for treating lung squamous cell carcinoma (Lung LUSC).

3. The use according to claim 1, **characterized in that** the SRE-PTPRZ1 inhibitors are the inhibitors of SRE-PTPRZ1 structural variation, which is a somatic simple repeat expansion (SRE) at insertion site GRCh38 chr7: 121603263-121603506, and the repeating pattern of an insertion region and a somatic SRE sequence are (CTTT)n, wherein 10 ≤ n < 500.

4. The use of PTPRZ1 inhibitors in the manufacture of medicaments for the treatment of SCC.

5. The use according to claim 4, **characterized in that** the medicaments are those for treating Lung LUSC.

6. The use according to claim 4, **characterized in that** the PTPRZ1 inhibitors are selected from the group consisting of NAZ2329, SCB4380, MY-33-3, MY-10, or a pharmaceutically acceptable salt thereof, or a derivative thereof, or a mixture thereof.

7. The use according to claim 4, **characterized in that** the PTPRZ1 inhibitors are the inhibitors of the PTPRZ1 gene, and the HGNC number of the PTPRZ1 gene is 9685.

8. A medicament for treating SCC, **characterized in that** it is formed by using SRE-PTPRZ 1 inhibitors or PTPRZ 1 inhibitors as the active ingredient, in combination with pharmaceutically acceptable excipients or auxiliary ingredients.

9. A medicament according to claim 8, **characterized in that** the medicament is that for treating Lung LUSC.

10. A medicament according to claim 8, **characterized in that** the SRE-PTPRZ1 inhibitors are the inhibitors of SRE-PTPRZ1 structural variation, which is a somatic SRE at the insertion site GRCh38 chr7: 121603263-121603506, and the repeating pattern of an insertion region and a somatic SRE sequence are (CTTT)n, wherein 10 ≤ n < 500.

11. A medicament according to claim 8, **characterized in that** the PTPRZ 1 inhibitors are selected from the group consisting of NAZ2329, SCB4380, MY-33-3, MY-10, or a pharmaceutically acceptable salt thereof, or a derivative thereof, or a mixture thereof.

12. A medicament according to claim 8, **characterized in that** the PTPRZ 1 inhibitors are the inhibitors of the PTPRZ1 gene, and the HGNC number of the PTPRZ1 gene is 9685.

13. The use of reagents for detecting the SRE-PTPRZ1 structural variation or the PTPRZ 1 gene in the manufacture of SCC screening kits.

14. The use according to claim 13, **characterized in that** the kit is a kit for screening Lung LUSC.

15. The use according to claim 13, **characterized in that** the reagents for detecting the SRE-PTPRZ1 structural variation or the PTPRZ1 gene is a PCR detection reagent.

16. The use according to claim 13, **characterized in that** the reagents for detecting the SRE-PTPRZ1 structural variation or the PTPRZ1 gene is a reagent for detecting the expression level of PTPRZ1 gene in tumor samples or blood samples.

17. The use according to claim 13, **characterized in that** the SRE-PTPRZ1 structural variation involves the somatic SRE at the insertion site GRCh38 chr7: 121603263-121603506, and the repeating pattern of an insertion region and a somatic SRE sequence are (CTTT)n, wherein 10 ≤ n < 500.

18. The use according to claim 13, **characterized in that** the HGNC number of the PTPRZ1 gene is 9685.

19. A screening kit for SCC, **characterized in that** it comprises reagents for detecting the SRE-PTPRZ1 structural variation or the PTPRZ1 gene.

20. The kit according to claim 19, **characterized in that** the kit is a kit for screening Lung LUSC.

21. The kit according to claim 19, **characterized in that** the reagents for detecting the SRE-PTPRZ1 structural variation or the PTPRZ1 gene is a PCR detection reagent.

22. The use according to claim 19, **characterized in that** the reagents for detecting the SRE-PTPRZ1 structural variation or the PTPRZ1 gene is a reagent for detecting the expression level of PTPRZ1 gene in tumor samples or blood samples.

23. The use according to claim 19, **characterized in that** the SRE-PTPRZ1 structural variation involves the somatic SRE at the insertion site GRCh38 chr7: 121603263-121603506, and the repeating pattern of an insertion region and a somatic SRE sequence are (CTTT)n, wherein 10 ≤ n < 500.

24. The kit according to claim 19, **characterized in that** the HGNC number of the PTPRZ1 gene is 9685.
